# EUROPÄISCHE PATENTSCHRIFT

(11) **EP 2 908 129 B1**
(45) Veröffentlichungstag und Bekanntmachung des Hinweises auf die Patenterteilung: **01.09.2021**
(21) Anmeldenummer: 15151302.5
(22) Anmeldetag: 30.05.2008
(51) Int. Cl.: G01N 33/487, A61B 5/145

(54) **SYSTEM ZUR MESSUNG EINER ANALYTKONZENTRATION EINER KÖRPERFLÜSSIGKEITSPROBE**
SYSTEM FOR MEASURING THE ANALYTE CONCENTRATION IN A BODY FLUID SAMPLE
SYSTÈME DE MESURE D'UNE CONCENTRATION D'ANALYSE D'UN ÉCHANTILLON DE FLUIDE CORPOREL

(30) Priorität: 15.06.2007 EP 07011740
(43) Veröffentlichungstag der Anmeldung: 19.08.2015
(62) Teilanmeldung aus: 08801442.8
(73) Patentinhaber: Roche Diabetes Care GmbH, 68305 Mannheim (DE); F. Hoffmann-La Roche AG, 4070 Basel (CH)
(72) Erfinder: Eisenhardt, Christoph, 68165 Mannheim (DE); Eikmeier, Heino, 64653 Lorsch (DE)
(74) Vertreter: Twelmeier Mommer & Partner

(56) Entgegenhaltungen:
- WO-A-96/13707
- WO-A-02/100261
- WO-A1-2006/069675
- DE-A1-102004 048 864
- US-A- 5 899 855
- US-B1- 6 602 469
- None

## Beschreibung

Die Erfindung betrifft ein Magazin für ein Handgerät zur Messung einer Analytkonzentration in einer Körperflüssigkeitsprobe mit dem im Oberbegriff des Anspruchs 1 angegebenen Merkmalen. Ein System mit einem Handgerät und einem derartigen Magazin ist beispielsweise aus der EP 1 574 855 A1 bekannt.

Zur Untersuchung von Urin, Blut, interstitieller Flüssigkeit oder anderen Körperflüssigkeiten wird üblicherweise Verbrauchsmaterial mit Nachweisreagenzien verwendet, die bei Kontakt mit einer Körperflüssigkeitsprobe eine Nachweisreaktion bewirken. Die Nachweisreaktion kann beispielsweise zu Fluoreszenz oder einer Farbänderung führen, die photometrisch zur Bestimmung einer Analytkonzentration ausgewertet werden kann. Bekannt sind beispielsweise auch Nachweisreaktionen zur elektrochemischen Bestimmung einer Analytkonzentration. Allgemein gesagt führt eine Nachweisreaktion zu einer Änderung eines physikalisch messbaren Parameters, wobei die Stärke der Änderung von der zu messenden Analytkonzentration abhängt. Typischerweise treten zwischen Produktionschargen von Verbrauchsmaterialien mit Nachweisreagenzien erhebliche Schwankungen der Nachweissensitivität auf. Damit zur Auswertung des Resultats einer Nachweisreaktion aus der Stärke der Änderung eines physikalischen Parameters, beispielsweise einer Verfärbung, mit einer für medizinische Anwendungen hinreichenden Genauigkeit eine Analytkonzentration ermittelt werden kann, sind deshalb Kalibrationsdaten erforderlich. Derartige Kalibrationsdaten werden üblicherweise für jede Produktionscharge mit Kalibrationsflüssigkeiten bekannter Analytkonzentration ermittelt und eine entsprechende Kalibrationsinformation auf einem Datenträger, der zusammen mit dem Verbrauchsmaterial vertrieben wird, gespeichert.

Bei Systemen zur Messung einer Analytkonzentration einer Körperflüssigkeitsprobe enthalten Handgeräte neben einer Messeinrichtung zum Messen des Resultats einer Nachweisreaktion deshalb üblicherweise eine Lesevorrichtung, um eine Kalibrationsinformation von einem Datenträger zu lesen. Bei dem aus der EP 1 574 855 A1 bekannten System ist auf der Außenseite eines das Verbrauchsmaterial enthaltenden Trommelmagazins ein Barcode angebracht, der eine Kalibrationsinformation enthält.

Ähnliche Messysteme zur Bestimmung einer Analytkonzentration in einer Körperflüssigkeitsprobe sind aus der DE102004048864A1 und der US2004038389A1 bekannt.

Das aus der EP 1 574 855 A1 bekannte System hat zwar den Vorteil, bei hohem Benutzerkomfort sicherzustellen, dass für das verwendete Verbrauchsmaterial stets die vom Hersteller vorgegebene Kalibrationsfunktion verwendet wird und folglich Bedienungsfehler ausgeschlossen sind, ist jedoch wenig anpassungsfähig.

Aufgabe der Erfindung ist es, ein Magazin der eingangs genannten Art zur Messung einer Analytkonzentration einer Körperflüssigkeitsprobe mit einem Handgerät weiter zu verbessern. Insbesondere soll ein Weg aufgezeigt werden, wie mit geringen Kosten auch von medizinischen Laien eine zuverlässige Messung einer Analytkonzentration einer Körperflüssigkeitsprobe durchgeführt werden kann.

Diese Aufgabe wird durch ein Magazin mit den im Anspruch 1 angegebenen Merkmalen gelöst. Vorteilhafte Weiterbildungen der Erfindung sind Gegenstand von Unteransprüchen.

Handgeräte von Systemen zur Messung einer Analytkonzentration in einer Körperflüssigkeitsprobe sind in der Herstellung kostenintensiv, da eine hochpräzise Messeinrichtung und ein Prozessor als Steuer- und Auswerteeinheit benötigt werden. Fortschritte bei der Herstellung von Verbrauchsmaterial mit Nachweisreagenzien können dazu führen, dass die bei Auslieferung eines Handgeräts zur Programmierung des Prozessors verwendete Algorithmen den Anforderungen verbesserter Verbrauchsmaterialen nicht mehr genügt, da beispielsweise andere Messparameter, beispielsweise andere Messzeiten, oder sogar andere Mess- oder Auswerteverfahren benötigt werden. Eine Änderung der Programmierung des Mikroprozessors ist bei dem aus der EP 1 574 855 A1 bekannten Handgerät jedoch nicht möglich, so dass Fortschritte bei der Messung der Analytkonzentration einer Körperflüssigkeitsprobe in der Regel die Anschaffung eines neuen Handgeräts erforderlich machen. Auch können eventuell vorhandene Fehler bei der Programmierung allenfalls durch eine aufwendige Rückrufaktion behoben werden.

Bei der erfindungsgemäßen Kombination von einem Magazin und einem Datenspeicher mit dem bekannten Messystem ist eine Anpassung an geänderte Anforderungen durch auf einem separaten Datenspeicher gespeicherte Ergänzungsdaten möglich. Die Ergänzungsdaten wirken beim Auswerten eines Messsignals mit einer auf dem Datenträger des in das Gerät eingelegten Magazins gespeicherten Kalibrationsinformation zusammen. Erfindungsgemäß enthalten die Ergänzungsdaten also von der Kalibrationsinformation verschiedene Daten, die zusammen mit der Kalibrationsinformation für eine korrekte Ermittlung einer Analytkonzentration zwingend erforderlich sind.

Bei einer Änderung des Verbrauchsmaterials lässt sich mit der Kalibrationsinformation alleine kein korrekter Analytkonzentrationswert ermitteln, da dafür zusätzlich auch die entsprechenden Ergänzungsdaten benötigt werden. Die Ergänzungsdaten können insbesondere für eine korrekte Berechnung einer Analytkonzentration aus einem Messsignal dienen und/oder den Messprozess selbst, also die korrekte Erzeugung des Messsignals, vorgeben. Bei den Ergänzungsdaten kann es sich beispielsweise um geänderte Mess- oder Auswerteparameter handeln, die durch eine Änderung des Verbrauchsmaterials bedingt sind. Ein erster Satz Ergänzungsdaten kann bei der Herstellung des Geräts in einem an dessen Prozessor angeschlossenen Speicher gespeichert werden. Geänderte Ergänzungsdaten können bei Bedarf von einem Datenspeicher eingelesen werden, der bevorzugt separat von dem Magazin ist.

Die unterschiedlichen Funktionen des Datenträgers, auf dem die Kalibrationsinformation gespeichert ist, und des Datenspeichers, auf dem Ergänzungsdaten gespeichert sind, bestehen somit darin, dass auf dem Datenträger chargenspezifische Daten gespeichert sind, also Daten, die sich typischerweise bei jeder Produktionscharge von Verbrauchsmaterial ändern, während die auf dem Datenspeicher gespeicherten Ergänzungsdaten für Verbrauchsmaterial mehrerer verschiedener Produktionschargen genutzt werden können. Die wichtigsten chargenspezifischen Daten sind Kalibrationsinformationen. Zusätzlich können auf dem Datenträger weitere chargenspezifische Daten, beispielsweise Herstellungsdatum oder Verfallsdatum, gespeichert sein.

Bei dem bekannten System kann mit Hilfe der erfindungsgemäßen Kombination
von einem Magazin und einem Datenspeicher sowie den gepeicherten
Daten die Software an geänderte Anforderungen angepasst werden. Die Ergänzungsdaten können neben geänderten Mess- oder Auswerteparametem also beispielsweise auch Softwareupdates enthalten, insbesondere für die Benutzerschnittstelle des Geräts bzw Menü- oder Bedienfunktionen, für eine Motorsteuerung, zur Bewertung gemessener Daten oder eine Schnittstelle zu externen Geräten. Derartige Ergänzungsdaten können zwar sehr umfangreich sein, werden in der Regel aber nur selten benötigt, so dass Datenspeicher mit Ergänzungsdaten ebenfalls nur selten benötigt werden. Die Kosten für Datenspeicher mit Ergänzungsdaten fallen deshalb in Relation zu den Kosten für Magazine mit Verbrauchsmaterial, die von einem typischen Benutzer in einer weit größeren Anzahl benötigt werden, nicht ins Gewicht.

Um die Programmierung eines Mikroprozessors bei Bedarf zu ändern, wurde bei der vorliegenden Erfindung nicht ein Weg eingeschlagen, auf dem an dem Magazin angebrachten Datenträger zusätzlich zur Kalibrationsinformation auch noch Ergänzungsdaten zu speichern, mit welchen die Programmierung des Mikroprozessors im Handgerät geändert werden könnte. Würden diese Daten auf dem am Magazin angebrachten Datenträger gespeichert, könnte zwar zuverlässig sichergestellt werden, dass dem Prozessor des Handgerätes stets die aktuell für Messungen mit dem Verbrauchsmaterial eines eingelegten Magazins benötigten Daten einschließlich Kalibrationsinformationen zur Verfügung steht. Auch wäre eine derartige Lösung benutzerfreundlich, da ein Benutzer zum Übertragen der Ergänzungsdaten in das Handgerät keine zusätzlichen Aktionen durchführen müsste.

Im Rahmen der vorliegenden Erfindung wurde jedoch erkannt, dass sich diese Vorteile mit einem System bestehend aus einem Handgerät und der erfindungsgemäßen Kombination von einem Magazin und einem Datenspeicher für das Handgerät wesentlich kostengünstiger erreichen lassen. Da Ergänzungsdaten wie beispielsweise Softwareupdates nur selten notwendig sind, würde eine Speicherung von Ergänzungsdaten auf dem Datenträger eines Magazins für jedes Magazin einen leistungsstarken und damit relativ teuren Speicher erforderlich machen, was zu spürbar höheren Kosten der in großen Stückzahlen produzierten Magazine führen würde. Erfindungsgemäß können diese höheren Magazinkosten dadurch vermieden werden, dass auf dem an dem Magazin angebrachten Datenträger lediglich ein relativ kleiner Datensatz, insbesondere die für jedes Magazin individuell benötigte Kalibrationsinformation, gespeichert wird, während ein größerer, sich seltener ändernder Datensatz mit Ergänzungsdaten auf einem separaten Datenspeicher gespeichert ist, der nur bei Bedarf, beispielsweise wenn Ergänzungsdaten wegen geändertem Verbrauchsmaterial erforderlich sind, ausgeliefert und im Handgerät ausgewechselt wird.

Die Erfindung macht bei einem Handgerät allenfalls minimale Zusatzkosten für eine Schnittstelle zum Übertragen von auf dem Datenspeicher gespeicherten Ergänzungsdaten auf das Handgerät erforderlich. Dem stehen erhebliche Kosteneinsparungen bei dem an dem Magazin angebrachten Datenträger gegenüber, der beispielsweise ebenso wie bei der EP 1 574 855 A1 kostengünstig als ein Barcode-Träger ausgestaltet werden kann.

Trotz der Aufteilung von Kalibrationsinformation und Ergänzungsdaten auf einen Datenträger am Magazin und auf einen austauschbaren Datenspeicher für das Handgerät kann bei einem erfindungsgemäßen System mit Sicherheit ausgeschlossen werden, dass Verbrauchsmaterial mit dafür nicht geeigneten Ergänzungsdaten verwendet wird, was zu fehlerhaften Messergebnissen und damit einer Gesundheitsgefährdung eines Benutzers führen könnte, wenn es nicht ausgeschlossen wäre. Erfindungsgemäß ist nämlich auf dem Datenträger zusammen mit der Kalibrationsinformation eine Verbrauchsmittelkennung gespeichert, die es dem Prozessor möglich macht zu prüfen, ob mit dem Verbrauchsmaterial des eingelegten Magazins in Kombination mit den dem Prozessor zur Verfügung stehenden Ergänzungsdaten eine zuverlässige Messung einer Analytkonzentration in einer Körperflüssigkeitsprobe möglich ist. Stellt der Mikroprozessor fest. dass dies nicht der Fall ist, beispielsweise weil ein benötigtes Update noch nicht vorgenommen wurde, wird ein Signal erzeugt, um einen Benutzer darauf aufmerksam zu machen.

Weitere Einzelheiten und Vorteile der Erfindung werden anhand eines Ausführungsbeispieles unter Bezugnahme auf die beigefügte Zeichnung erläutert. Die dabei beschriebenen Merkmale können einzeln und in Kombination zum Gegenstand von Ansprüchen gemacht werden. Es zeigen:
- Figur 1: eine schematische Darstellung eines Ausführungsbeispieles eines Systems zur Messung einer Analytkonzentration einer Körperflüssigkeitsprobe;
- Figur 2: ein austauschbares Magazin mit Verbrauchsmaterial;
- Figur 3: ein Handgerät, in welches das in Figur 2 gezeigte Magazin eingelegt werden kann,
- Figur 4: einen austauschbaren Datenspeicher für das in Figur 3 gezeigte Handgerät; und
- Figur 5: eine schematische Darstellung der Komponenten des in Figur 3 gezeigten Handgeräts.

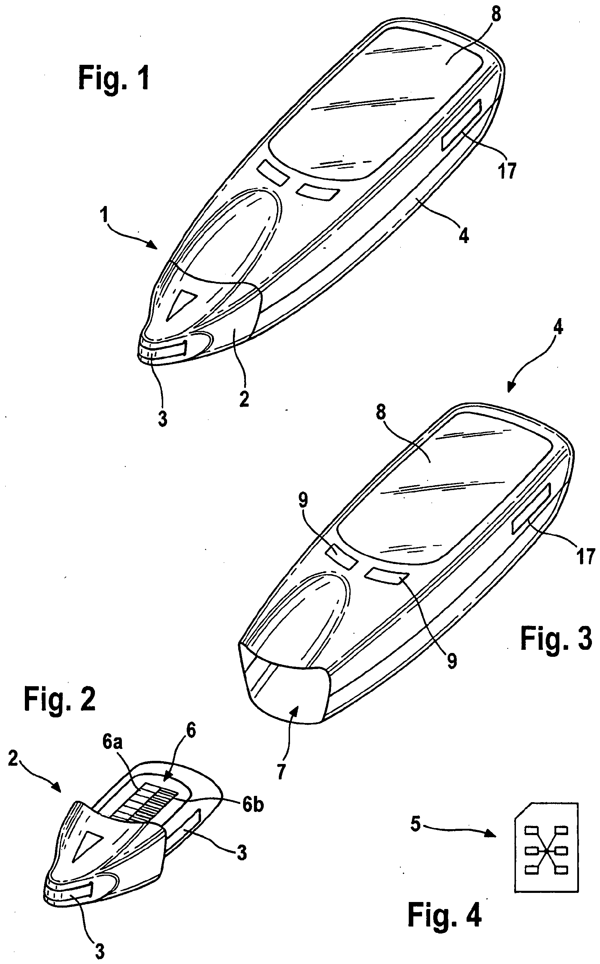

Figur 2 zeigt ein System 1 zur Messung einer Analytkonzentration in einer Körperflüssigkeitsprobe, beispielsweise zur Messung der Glukosekonzentration von Blut und/oder interstitieller Flüssigkeit. Derartige Systeme werden beispielsweise von Diabetikern benötigt, die mehrmals täglich ihren Blutzuckergehalt messen müssen. Das dargestellte System 1 umfasst mindestens drei separate Systemkomponenten, nämlich ein in Figur 2 gezeigtes Magazin 2 mit Verbrauchsmaterial 3, ein in Figur 3 gezeigtes Handgerät 4, das bestimmungsgemäß das Magazin 2 aufnimmt, sowie einen austauschbaren Datenspeicher 5 mit Ergänzungsdaten für das Handgerät 4.

Diese Systemkomponenten 2, 4, 5 werden im Folgenden unter Bezugnahme auf die dazugehörenden Figuren näher erläutert.

Figur 2 zeigt ein Ausführungsbeispiel eines Magazins 2, das Verbrauchsmaterial 3 für mehrere Messungen enthält. Das Verbrauchsmaterial 3 ist bei dem dargestellten Ausführungsbeispiel ein Band, das Nachweisreagenzien trägt, die bei Kontakt mit einer Körperflüssigkeitsprobe eine Nachweisreaktion bewirken. Die Nachweisreaktion führt zu einer Verfärbung des mit der Körperflüssigkeitsprobe benetzten Bandabschnitts. Diese Verfärbung kann photometrisch ausgewertet werden, um die Analytkonzentration der aufgebrachten Körperflüssigkeitsprobe, bei dem dargestellten Ausführungsbeispiel, also die Glukosekonzentration, zu bestimmen. Bei dem dargestellten Ausführungsbeispiel ist das Band 3 kontinuierlich mit Nachweisreagenzien versehen, z. B. bedeckt oder imprägniert. Möglich ist es auch die Nachweisreagenzien nur in einzelnen Testfeldern auf das Band 3 aufzubringen. Insbesondere können zwischen derartigen Testfeldern auf dem Band Lanzetten angeordnet werden, um ein Magazin für ein Handgerät zu schaffen, mit dem eine Stichwunde zur Gewinnung einer Körperflüssigkeitsprobe erzeugt und anschließend eine Untersuchung der gewonnenen Körperflüssigkeitsprobe durchgeführt werden kann.

Das Band 3 ist in dem Magazin 2 auf einer Vorratsrolle (nicht dargestellt) aufgewickelt, kann jedoch beispielsweise auch zick-zack-förmig zu einem Stapel gefaltet sein. Das Magazin enthält eine antreibbare Förderrolle (nicht dargestellt), auf die der gebrauchte Abschnitt des Bands 3 aufgewickelt wird. Durch Drehen der Förderrolle kann ein frischer, d. h. unbenutzter Bandabschnitt in eine Probenaufnahmeposition bewegt werden, in der er mit einer Körperflüssigkeitsprobe benetzt werden kann. Durch weitere Drehung der Förderrolle kann ein Bandabschnitt mit einer aufgenommenen Körperflüssigkeitsprobe in eine Messposition gefördert und ein frischer Bandabschnitt für eine weitere Messung in die Probenaufnahmeposition bewegt werden.

An dem Magazin 2 ist ein Datenträger 6 angebracht, der im dargestellten Ausführungsbeispiel durch ein Druckverfahren hergestellt ist und die Daten beispielsweise als Barcode trägt. Möglich ist es beispielsweise auch, den Datenträger 6 als einen OTP (one time programmable) oder magnetischen Speicher auszubilden. Möglich ist es beispielsweise auch den Datenträger 6 als einen gedruckten elektronischen Speicher auszubilden, der von der Lesevorrichtung 10 des Handgeräts 4 zum Auslesen kontaktiert wird. Beispielsweise kann der Datenträger 6 als ein Array von Widerständen ausgebildet sein, wobei die auf ihm gespeicherten Daten durch unterschiedliche Widerstandswerte kodiert sind. Möglich ist es auch, dass der Datenträger 6 unterschiedliche Farbflächen trägt, mit denen Daten codiert sind.

Der Datenträger 6 enthält eine Kalibrationsinformation für das in dem Magazin 2 enthaltene Verbrauchsmaterial 3. Der Barcode wird beim Einschieben des Magazins 2 in ein Magazinaufnahmefach 7 eines zu dem System 1 gehörenden Handgeräts 4 gelesen. Die die Geschwindigkeit des Einschiebens von Benutzer zu Benutzer verschieden ist, hat der dargestellte Datenträger 6 eine Taktspur 6a mit äquidistanten Taktmarken und eine Informationsspur 6b, welche die Kalibrationsinformation enthält. Andere technische Lösungen zum Einlesen eines Barcodes sind auch möglich, insbesondere lassen sich vorteilhaft auch Barcodeleser nutzen, die keine Relativbewegung zwischen dem Handgerät 4 und dem Datenträger erfordern, also einen gegenüber dem Handgerät 4 ruhenden Datenträger 6 einlesen können. Derartige Barcodeleser können beispielsweise mit einer CCD-Zeile ausgestattet sein, so dass der gesamte Barcode ohne Relativbewegung erfasst werden kann.

Bei dem dargestellten Ausführungsbeispiel ist das Magazin 2 mit dem Verbrauchsmaterial 3 als eine Bandkassette ausgebildet. Möglich ist es aber auch, das Magazin als ein Trommelmagazin auszubilden, wie es beispielsweise aus der EP 1 574 855 A1 bekannt ist.

Das in Figur 2 dargestellte Magazin 2 kann in ein Magazinaufnahmefach 7 des in Figur 3 dargestellten Handgeräts 4 eingesetzt werden. Das dargestellte Handgerät 4 hat etwa die Größe eines Mobiltelefons und kann deshalb von einem Benutzter problemlos in einer Jackentasche mitgeführt werden. Das Handgerät 4 kann netzunabhängig betrieben werden, beispielsweise mittels Batterien. Messergebnisse können mit einer Anzeigeeinrichtung 8, beispielsweise mit einer Flüssigkristallanzeige, angezeigt werden. Zu seiner Bedienung weist das Handgerät 4 ferner Bedienungselemente 9 auf, die bei dem dargestellten Ausführungsbeispiel als Tasten ausgebildet sind.

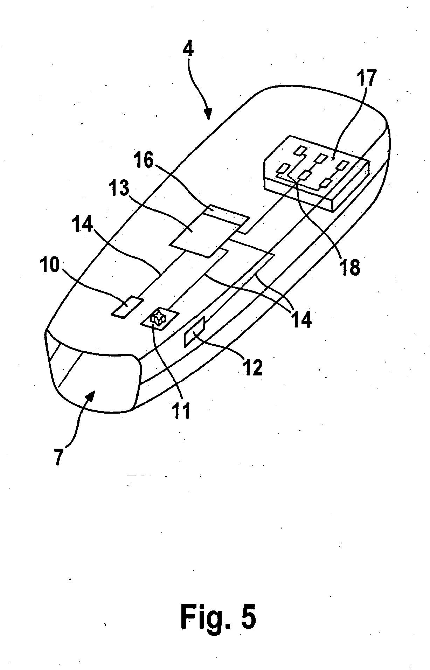

Der innere Aufbau des in Figur 3 gezeigten Handgeräts 4 ist schematisch in Figur 5 dargestellt, auf die im Folgenden ebenfalls Bezug genommen wird. Das Handgerät 4 hat ein Magazinaufnahmefach 7 mit einer Lesevorrichtung 10, um ein in Figur 2 dargestelltes Magazin 2 aufzunehmen und einen daran befestigten Datenträger 6 zu lesen. Mit einer Transporteinrichtung 11 kann Verbrauchsmaterial 3 eines in das Magazinaufnahmefach 7 eingelegten Magazins 2 für eine Probenaufnahme und eine Messung der Analytkonzentration in einer aufgenommenen Körperflüssigkeitsprobe bewegt werden. Die Transporteinrichtung 11 kann beispielsweise ähnlich wie bei einem Tonbandgerät zum Transport des Bandes 3 ausgebildet sein. Die Transporteinrichtung könnte zur Vermeidung von Schlupf auch ein Indexrad mit Zähnen aufweisen, die in eine fortlaufende Perforation des Bandes 3 eingreifen. Bei dem dargestellten Ausführungsbeispiel umfasst die Transporteinrichtung 11 gemäß Figur 5 eine Welle mit einem von der Kreisform abweichenden Kopf, beispielsweise mit einem sternförmigen Kopf, der in eine dazu passende Ausnehmung der antreibbaren Förderrolle des in Figur 2 dargestellten Magazins 2 eingreift. Nach Aufnahme einer Probe kann das Band 3 mit der Transporteinrichtung 11 weiterbewegt werden, so dass ein mit der Probe benetzter Bandabschnitt in eine Messposition bewegt wird, in der mit einer Messeinrichtung 12 des Handgeräts 4 das Resultat einer Nachweisreaktion, beispielsweise der Grad einer durch einen nachzuweisenden Analyten bewirkten Verfärbung, gemessen wird.

Die Transporteinrichtung 11, die Messeinrichtung 12 und die Lesevorrichtung 10 werden von einem Prozessor 13 gesteuert, an den sie über Datenleitungen 14 angeschlossen sind. Der Prozessor 13 wertet von der Messeinrichtung 12 gemessene Messsignale aus, um einen Konzentrationswert zu ermitteln, der mit der Anzeigeeinrichtung 8 angezeigt wird. An den Prozessor 13 ist ein Speicher 16 angeschlossen, in dem Software für den Prozessor 13 gespeichert ist. Der Prozessor 13 und sein Speicher 16 können Bestandteil eines Mikrokontrollers oder Mikrocomputers sein.

Zu dem in Figur 1 dargestellten System gehört als weitere separate Systemkomponente ein austauschbarer Datenspeicher 5, der in Figur 4 dargestellt ist und in ein Aufnahmefach 17 des in Figur 3 dargestellten Handgeräts 4 eingelegt werden kann. Das Aufnahmefach 17 ist durch einen Schlitz in dem Gehäuse des Handgeräts 4 zugänglich.

Auf dem Datenspeicher 5 gespeicherte Daten können über eine Schnittstelle des Handgeräts 4 auf das Handgerät übertragen werden. Bei dem dargestellten Ausführungsbeispiel ist in der Schnittstelle eine Leseeinrichtung 18 enthalten, um einen in das Aufnahmefach 17 eingelegten Datenspeicher 5 zu lesen.

Der austauschbare Datenspeicher 5 kann beispielsweise ein EEPROM, insbesondere ein Flash-EEPROM, eine Smartcard oder ein sonstiger Speicherchip sein. Prinzipiell ist es auch möglich, den austauschbaren Datenspeicher als einen RFID-Speicher auszubilden und Daten über eine drahtlose Schnittstelle auf das Handgerät 4 zu übertragen, so dass auf ein Aufnahmefach für den Datenspeicher 5 verzichtet werden kann. Möglich ist es auch, dass der Hersteller bei Bedarf den Datenspeicher 5 an dem Magazin 2 selbst befestigt, so dass kein separates Aufnahmefach benötigt wird.

Auf dem Datenspeicher 5 sind Ergänzungsdaten gespeichert, die beim Auswerten eines Messsignals mit einer auf dem Datenträger 6 eines in das Gerät 4 eingelegten Magazins 2 gespeicherten Kalibrationsinformation und der auf dem Speicher 16 des Handgeräts 4 gespeicherten Software zusammenwirken. Die Ergänzungsdaten können beispielsweise Mess- oder Belichtungszeiten zur Steuerung der Messeinrichtung 12 festlegen. Mittels des Datenspeichers 5 ist es für den Systemanbieter möglich, Mess- oder Auswertealgorithmen nach Bedarf abzuändern oder zu ersetzten, und so das Handgerät 4 an geänderte Anforderungen, die sich beispielsweise durch verbessertes Verbrauchsmaterial 3 ergeben können, anzupassen. Zusätzlich zu Daten, die für eine zusammen mit der Kalibrationsinformation für eine korrekte Ermittlung einer Analytkonzentration zwingend erforderlich sind, können die Ergänzungsdaten also auch herkömmlich Softwareupdates, beispielsweise zur Änderung der Benutzeroberfläche, enthalten.

Auf dem austauschbaren Datenspeicher 5 gespeicherte Ergänzungsdaten werden vorzugsweise in den Speicher 16 des Handgeräts 4 kopiert, so dass der Datenspeicher 5 nach dem Auslesen aus dem Aufnahmefach 17 entnommen werden kann. Die Ergänzungsdaten können nach dem Auslesen auf dem Datenträger 5 gelöscht werden, um Missbrauch zu erschweren. Möglich ist es aber auch, von einem Speichern der Ergänzungsdaten auf dem Speicher 16 des Handgeräts abzusehen, so dass das Handgerät 5 nur mit einem eingelegten Datenspeicher 5 betrieben werden kann.

Auf dem Datenträger 6 des in Figur 2 dargestellten Magazins 2 ist eine Verbrauchsmittelkennung gespeichert, mit welcher der Prozessor 13 nach dem Einlegen des Magazins 2 ermittelt, ob mit dem Verbrauchsmaterial 3 des eingelegten Magazins 2 in Kombination mit den Ergänzungsdaten, welche dem Prozessor 13 zur Verfügung stehen, eine zuverlässige Messung einer Analytkonzentration in einer Körperflüssigkeitsprobe möglich ist. Falls dies der Fall ist, kann eine Messung der Analytkonzentration in einer Körperflüssigkeitsprobe durchgeführt werden. Falls dies nicht der Fall ist, erzeugt der Prozessor 13 ein Signal, um einem Benutzer zu signalisieren, dass mit den zur Verfügung stehenden Ergänzungsdaten und dem Verbrauchsmaterial 3 des eingelegten Magazins 2 keine gültige Messung einer Analytkonzentration in einer Körperflüssigkeitsprobe vorgenommen werden kann. Das von dem Prozessor 13 zu diesem Zweck erzeugte Signal kann irgendeine Aktion des Handgeräts 4 bewirken, die einen Benutzer erkennen lässt, dass mit den zur Verfügung stehenden Ergänzungsdaten und dem Verbrauchsmaterial 3 des eingelegten Magazins 2 keine gültige Messung einer Analytkonzentration in einer Körperflüssigkeitsprobe vorgenommen werden kann. Das Signal des Prozessors 13 kann beispielsweise die Anzeigeinrichtung 8 betätigen, um eine entsprechende Mitteilung anzuzeigen und/oder eine akustische Signaleinrichtung zum Erzeugen eines akustischen Signals veranlassen.

Auf diese Weise kann zuverlässig erreicht werden, dass Verbrauchsmaterial 3 des Systems 1 stets nur in Kombination mit den vom Hersteller dafür vorgesehenen Ergänzungsdaten, insbesondere dafür geeignete und aktuelle Algorithmen, verwendet wird. Insbesondere für den Fall, dass die Ergänzungsdaten eine Änderung der Algorithmen bewirken, die in dem Speicher 16 des Handgeräts 4 gespeichert ist, kann nämlich das Durchführen und Auswerten einer Messung mit Verbrauchsmaterial 3, das andere Ergänzungsdaten voraussetzt, zu falschen Messergebnissen führen. Ein entsprechendes Signal kann dem Benutzer beispielsweise mittels der Anzeigeeinrichtung 8 angezeigt werden. Geeignet sind insbesondere auch akustische Signale, beispielsweise Pieptöne. Besonders günstig ist es, das Warnsignal, dass mit den zur Verfügung stehenden Systemkomponenten 3, 4, 5 keine gültige Messung vorgenommen werden kann, sowohl akustisch als auch optisch zu übertragen.

Auf dem an dem Magazin 2 befestigten Datenträger 6 können neben der Kalibrationsinformation weitere chargenspezifische Daten gespeichert sein, die das Verbrauchsmaterial einer Produktionscharge charakterisieren. Verbrauchsmaterial 3 mit Nachweisreagenzien wird typischerweise chargenweise produziert, wobei sich die einzelnen Produktionschargen wegen unvermeidbarer Variationen des Herstellungsprozesses hinsichtlich ihrer Sensitivität unterscheiden, so dass zur Auswertung von mit dem Verbrauchsmaterial 3 vorgenommenen Messungen eine Kalibrationsinformation benötigt wird. Die Kalibrationsinformation kann explizit die Sensitivität des Verbrauchsmaterials 3 angeben und beispielsweise für einen oder mehrere Stützwerte, aus denen eine Auswertekurve gebildet werden kann, die Stärke der mit der Nachweisreaktion verbundenen Parameteränderung, beispielsweise den Grad der photometrisch auszuwertenden Verfärbung, für eine oder mehrere Analytkonzentrationen explizit angeben. Möglich ist es auch, dass in dem Speicher 16 des Handgeräts eine Bibliothek von Auswertekurven gespeichert ist und die Kalibrationsinformation lediglich die Nummer der zu verwendenden Auswertekurve angibt.

Neben der Kailibrationsinformation ist beispielsweise das Herstellungsdatum und/oder ein Verfallsdatum eine weitere chargenspezifische Information. Indem auf dem Datenträger 6 eine Information gespeichert ist, aus welcher der Prozessor 13 ein Verfallsdatum für das in dem Magazin 2 enthaltene Verbrauchsmaterial 3 ermitteln kann, lässt sich zudem ausschließen, dass durch Verwendung von altem und deshalb unzuverlässigem Verbrauchsmaterial fehlerhafte Messergebnisse gewonnen werden.

Der Prozessor 13 des dargestellten Ausführungsbeispiels überprüft deshalb nach Einlegen eines Magazins 2 in das Magazinaufnahmefach 7 des Handgeräts 4 nicht nur, ob die Kalibrationsinformation zu den zur Verfügung stehenden Ergänzungsdaten passt, sondern auch, ob ein Verfallsdatum für das Verbrauchsmaterial für das eingelegte Magazin bereits überschritten ist. Falls dies der Fall ist, wird dies einem Benutzter ebenfalls signalisiert.

Während der Datenträger 6 des dargestellten Ausführungsbeispiels alle für eine Messung benötigten chargenspezifischen Daten enthält, sind auf dem Datenspeicher 5 bevorzugt ausschließlich Daten gespeichert, die für Messungen mit Verbrauchsmaterial 3 aus mehreren, gegebenenfalls auch aus unterschiedlichen Magazinen 2 verwendet werden können. Der Datenspeicher 5 enthält also bevorzugt ausschließlich chargenunspezifische Daten, die ohne Änderungen für mehrere Magazine verwendet werden können. Erst wenn die Notwendigkeit für geänderte Ergänzungsdaten aufgetreten ist, beispielsweise zur Anpassung an verbessertes Verbrauchsmaterial 3 öder zur Behebung von erkannten Schwächen der Software, werden neu produzierte Magazine 2 mit einer neuen Kennung versehen, so dass das Handgerät 4 einen Benutzer auf die Notwendigkeit von neuen Ergänzungsdaten hinweist, wenn es die Kennung eines neuen Magazins noch nicht kennt.

Auf dem Datenträger 6 sind typischerweise weniger als 200 Bit gespeichert, da dies für eine Kennung, eine Kalibrationsinformation und andere chargenspezifische Informationen im Allgemeinen ausreicht. Der Datenspeicher 5 hat bei dem gezeigten Ausführungsbeispiel ein größeres Speichervolumen als der Datenträger 6. Im Allgemeinen sind auf dem Datenspeicher 5 mehr als 500 Bit, insbesondere mehr als 1 kBit gespeichert. Bei umfangreichen Softwareupdates können auf dem Datenspeicher 5 auch wesentlich größere Datenmengen gespeichert sein.

### Bezugszahlen

- 1: Messsystem
- 2: Magazin
- 3: Verbrauchsmaterial mit Nachweisreagenzien
- 4: Handgerät
- 5: Datenspeicher
- 6: Datenträger
- 6a: Taktspur
- 6b: Informationsspur
- 7: Magazinaufnahmefach
- 8: Anzeigeeinrichtung
- 9: Bedienungselemente
- 10: Lesevorrichtung
- 11: Transporteinrichtung
- 12: Messeinrichtung
- 13: Prozessor
- 14: Datenleitung

- 16: Speicher
- 17: Aufnahmefach
- 18: Schnittstelle mit Leseeinrichtung

## Patentansprüche

1. Magazin mit einem Datenspeicher (5) für ein Handgerät (4) zur Messung einer Analytkonzentration in einer Körperflüssigkeitsprobe, wobei das Handgerät (4) eine Schnittstelle (18) aufweist und das Magazin (2) Verbrauchsmaterial (3) für mehrere Messungen mit Nachweisreagenzien, die bei Kontakt mit der Körperflüssigkeitsprobe eine Nachweisreaktion bewirken, enthält, wobei der Datenspeicher (5) für das Handgerät (4) austauschbar ist,
**dadurch gekennzeichnet, dass**
an dem Magazin (2) ein Datenträger (6) angebracht ist, der eine Kalibrationsinformation für das in dem Magazin (2) enthaltene Verbrauchsmaterial (3) enthält,
auf dem Datenspeicher (5) Ergänzungsdaten gespeichert sind, die über Schnittstelle (18) auf das Handgerät (4) übertragen werden, und
die beim Auswerten eines Messsignals mit einer auf dem Datenträger (6) des in das Gerät (4) eingelegten Magazins (2) gespeicherten Kalibrationsinformation zusammenwirken, und
auf dem Datenträger (6) eine Verbrauchsmittelkennung gespeichert ist, mit welcher der Prozessor (13) nach Einlegen des Magazins (2) in das Handgerät (4) ermittelt, ob die Kalibrationsinformation des an dem Magazin (2) angebrachten Datenträgers (6) zu den auf dem Datenspeicher (5) gespeicherten Ergänzungsdaten passt und so ermittelt werden kann, ob mit dem Verbrauchsmaterial (3) des Magazins (2) in Kombination mit den zur Verfügung stehenden Ergänzungsdaten eine zuverlässige Messung einer Analytkonzentration in der Körperflüssigkeitsprobe möglich ist.

2. Magazin nach Anspruch 1, **dadurch gekennzeichnet, dass** der Datenträger (6) an dem Magazin (2) befestigt ist.

3. Magazin nach einem der vorstehenden Ansprüche, **dadurch gekennzeichnet, dass** auf dem Datenträger (6) neben der Kalibrationsinformation weitere chargenspezifischen Daten gespeichert sind, die Verbrauchsmaterial (3) einer Produktionscharge charakterisieren.

4. Magazin nach einem der vorhergehenden Ansprüche, **dadurch gekennzeichnet, dass** auf dem Datenspeicher (5) ausschließlich Daten gespeichert sind, die für Messungen mit Verbrauchsmaterial (3) aus mehreren Magazinen (2) verwendet werden können.

5. Magazin nach einem der vorhergehenden Ansprüche, **dadurch gekennzeichnet, dass** auf dem Datenträger (6) weniger als 200 Bit gespeichert sind.

6. Magazin nach einem der vorhergehenden Ansprüche, **dadurch gekennzeichnet, dass** auf dem Datenspeicher (5) mehr als 500 Bit, vorzugsweise mehr als 1 kBit, gespeichert sind.

7. Magazin nach einem der vorhergehenden Ansprüche, **dadurch gekennzeichnet, dass** der Datenträger (6) einen Barcode trägt oder ein magnetischer Speicher ist.

8. Magazin nach einem der vorhergehenden Ansprüche, **dadurch gekennzeichnet, dass** der Datenspeicher (5) ein EEPROM ist.

## Claims

1. Cartridge (2) comprising a data storage unit (5) for a hand-held device (4) for measuring an analyte concentration in a body fluid sample, wherein the hand-held device (4) has an interface (18) and the cartridge (2) contains consumables (3) for several measurements with detection reagents that effect a detection reaction when exposed to the body fluid sample, wherein the data storage unit (5) for the hand-held device (4) is exchangeable,
**characterized in that**
a data carrier (6) is attached to the cartridge (2) and contains a calibration information for the consumables (3) contained in the cartridge (2),
supplementary data is stored in the data storage unit (5) which is transmitted via the interface (18) to the hand-held device (4) and during the analysis of a measuring signal interacts with a calibration information that is stored on the data carrier (6) of the cartridge (2) that is inserted in the device (4), and
a consumables identification is stored on the data carrier (6), which is used by the processor (13) after insertion of the cartridge (2) into the hand-held device (4) to determine whether the calibration information of the data carrier (6). which is attached to the cartridge (2), matches the supplementary data stored in the data storage unit (5) and it is thereby possible to determine whether a reliable measurement of an analyte concentration in the body fluid sample is feasible by means of the consumables (3) of the inserted cartridge (2) combined with the supplementary data.

2. Cartridge according to claim 1, **characterized in that** the data carrier (6) is fixed to the cartridge.

3. Cartridge according to any one of the preceding claims, **characterized in that**, aside from the calibration information, further batch-specific data characterizing the consumables (3) of a production batch is stored on the data carrier (6).

4. Cartridge according to any one of the preceding claims, **characterized in that** the data storage unit (5) exclusively stores data that can be used for measurements using consumables (3) from multiple cartridges (2).

5. Cartridge according to any one of the preceding claims, **characterized in that** less than 200 bit are stored on the data carrier (6).

6. Cartridge according to any one of the preceding claims, **characterized in that** in excess of 500 bit, preferably in excess of 1 kbit, are stored in the data storage unit (5).

7. Cartridge according to any one of the preceding claims, **characterized in that** the data carrier (6) carries a bar code or is a magnetic memory.

8. Cartridge according to any one of the preceding claims, **characterized in that** the data storage unit (5) is an EEPROM.

## Revendications

1. Magasin avec une mémoire de données (5), destiné à un appareil portable (4) de mesure d'une concentration d'analyte dans un échantillon de fluide corporel, l'appareil portable (4) comportant une interface (18) et le magasin (2) contenant de la matière consommable (3) pour plusieurs mesures à l'aide de réactifs de détection, qui en cas de contact avec l'échantillon de fluide corporel provoquent une réaction de détection, la mémoire de données (5) pour l'appareil portable (4) étant interchangeable,
**caractérisé en ce que**
sur le magasin (2) est monté un support de données (6) qui contient une information de calibrage pour la matière consommable (3) contenue dans le magasin (2),
dans la mémoire de données (5) sont mémorisées des données complémentaires, qui par l'intermédiaire de l'interface (18), sont transférées à l'appareil portable (4) et qui lors de l'évaluation d'un signal de mesure, coopèrent avec une information de calibrage mémorisée sur le support de données (6) du magasin (2) inséré dans l'appareil (4), et
sur le support de données (6) est mémorisée une identification de matière consommable, à l'aide de laquelle, après l'insertion du magasin (2) dans l'appareil portable (4), le processeur (13) détermine si l'information de calibrage du support de données (6) monté sur le magasin est adaptée aux données complémentaires mémorisées dans la mémoire de données (5) et il peut ainsi être déterminé si à l'aide de la matière consommable (3) du magasin (2), en association avec les données complémentaires disponibles, il est possible de procéder à une mesure fiable d'une concentration d'analyte dans l'échantillon de fluide corporel.

2. Magasin selon la revendication 1, **caractérisé en ce que** le support de données (6) est fixé sur le magasin (2).

3. Magasin selon l'une quelconque des revendications précédentes, **caractérisé en ce que** sur le support de données (6) sont mémorisées parallèlement à l'information de calibrage d'autres données spécifiques à la charge, qui caractérisent une matière consommable (3) d'une charge de production.

4. Magasin selon l'une quelconque des revendications précédentes, **caractérisé en ce que** dans la mémoire de données (5) sont mémorisées exclusivement des données susceptibles d'être utilisées pour procéder à des mesures avec de la matière consommable (3) provenant de plusieurs magasins (2).

5. Magasin selon l'une quelconque des revendications précédentes, **caractérisé en ce que** dans le support de données (6) sont mémorisés moins de 200 bits.

6. Magasin selon l'une quelconque des revendications précédentes, **caractérisé en ce que** dans la mémoire de données (5) sont mémorisés plus de 500 bits, de préférence est mémorisé plus de 1 kbit.

7. Magasin selon l'une quelconque des revendications précédentes, **caractérisé en ce que** le support de données (6) porte un code à barres ou est une mémoire magnétique.

8. Magasin selon l'une quelconque des revendications précédentes, **caractérisé en ce que** la mémoire de données (5) est une EEPROM.
